# EUROPÄISCHE PATENTSCHRIFT

(11) **EP 1 155 018 B1**
(45) Veröffentlichungstag und Bekanntmachung des Hinweises auf die Patenterteilung: **30.10.2002**
(21) Anmeldenummer: 00909166.1
(22) Anmeldetag: 14.02.2000
(51) Int. Cl.: C07D 471/04, A61K 31/4709, A61P 31/04

(54) **KRISTALLMODIFIKATION C VON 8-CYAN-1-CYCLOPROPYL-7-(1S,6S-2,8DIAZABICYCLO-(4.3.0) NONAN-8-YL)-6-FLUOR-1,4-DIHYDRO-4-OXO-3-CHINOLINCARBONSAURE**
CRYSTAL MODIFICATION C OF 8-CYANO-1-CYCLOPROPYL-7- (1S, 6S-2,8- DIAZABICYCLO - 4.3.0] NONAN-8-YL) -6-FLUORO-1,4-DIHYDRO-4-OXO-3-QUINOLINE CARBOXYLIC
MODIFICATION CRISTALLINE C DE L'ACIDE 8-CYANO -1- CYCLOPROPYL -7-(1S, 6S-2,8- DIAZABICYCLO - 4.3.0] NONAN -8-YL) -6-FLUORO -1,4- DIHYDRO -4-OXO -3-QUINOLEINE -CARBOXYLIQUE

(30) Priorität: 26.02.1999 DE 19908449
(43) Veröffentlichungstag der Anmeldung: 21.11.2001
(73) Patentinhaber: Bayer Aktiengesellschaft, 51368 Leverkusen (DE)
(72) Erfinder: RAST, Hubert, D-51381 Leverkusen (DE); HIMMLER, Thomas, D-51519 Odenthal (DE)
(86) Internationale Anmeldenummer: EP0001202
(87) Internationale Veröffentlichungsnummer: WO00052009

(56) Entgegenhaltungen:
- WO-A-97/31001
- DE-A- 19 546 249

## Beschreibung

Die vorliegende Erfindung betrifft eine definierte Kristallmodifikation von 8-Cyan-1-cyclopropyl-7-(1 S,6S-2,8-diazabicyclo[4.3.0]nonan-8-yl)-6-fluor-1,4-dihydro-4-oxo-3-chinolincarbonsäure, Verfahren zu ihrer Herstellung und ihre Verwendung in pharmazeutischen Zubereitungen.

8-Cyan-1-cyclopropyl-7-(1S,6S-2,8-diazabicyclo[4.3.0]nonan-8-yl)-6-fluor-1,4-dihydro-4-oxo-3-chinolincarbonsäure der Formel (I) soll im folgenden als CCDC bezeichnet werden.

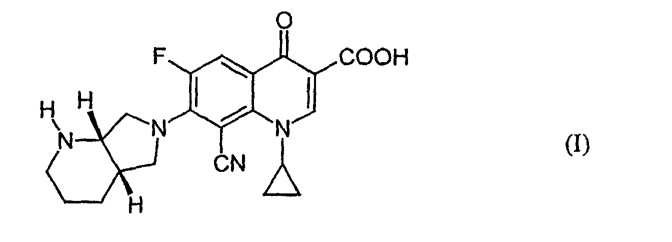

CCDC ist bekannt aus DE-A 19 633 805 oder PCT Anm.-Nr. 97 903 260.4. Sie wird danach hergestellt durch Umsetzung von 7-Chlor-8-cyan-1-cyclopropyl-6-fluor-1,4-dihydro-4-oxo-3-chinolincarbonsäure mit (1S,6S)-2,8-Diazabicyclo[4.3.0]nonan in einem Gemisch aus Dimethylformamid und Acetonitril in Gegenwart einer Hilfsbase. Nach Versetzen mit Wasser wird CCDC mit Dichlormethan aus Wasser extrahiert und durch Entfernen des Extraktionsmittels isoliert. Man erhält dabei ein Pulver, das keine eindeutige Kristallmodifikation aufweist. Das Pulver ist vielmehr zu großen Teilen amorph und kann Gemische verschiedener Kristallmodifikationen enthalten. Sollte durch Zufall eine einheitliche Kristallmodifikation entstehen ist unklar, wie sie extrahiert und definiert erhalten werden kann. Für die Herstellung von Arzneimitteln ist jedoch Voraussetzung, dass für einen Wirkstoff, der in verschiedenen Kristallmodifikationen vorliegen kann, eindeutig angegeben wird, in welcher Kristallmodifikation er zur Herstellung des Mittels eingesetzt wird.

Das z.T. amorphe Pulver, das nach dem oben skizzierten Herstellverfahren erhalten wird, ist zudem hygroskopisch. Amorphe Feststoffe, und besonders hygroskopische Feststoffe, sind in der galenischen Verarbeitung schlecht handzuhaben, da sie beispielsweise geringe Schüttdichten und mangelhafte Fließeigenschaften aufweisen können. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehaltes oder der Stabilität in den produzierten Festformulierungen zu erhalten.

Der Erfindung liegt daher die Aufgabe zugrunde, eine kristallinc Form definierter Modifikation von CCDC herzustellen, die aufgrund ihrer physikalischen Eigenschaften, insbesondere ihrer Kristalleigenschaften in galenischen Formulierungen gut zu handhaben ist.

Diese Aufgabe wird erfindungsgemäß durch eine neue, kristalline Form von CCDC gelöst, die nachstehend als Modifikation C bezeichnet wird.

Gegenstand der Erfindung ist daher die kristalline Modifikation C von CCDC, die dadurch gekennzeichnet ist, dass sie ein Röntgen-Pulverdiffraktogramm mit den in der folgenden Tabelle 1 angegebene Reflexlagen (2 Theta) hoher und mittlerer Intensität (> 15% relative Intensität) aufweist.

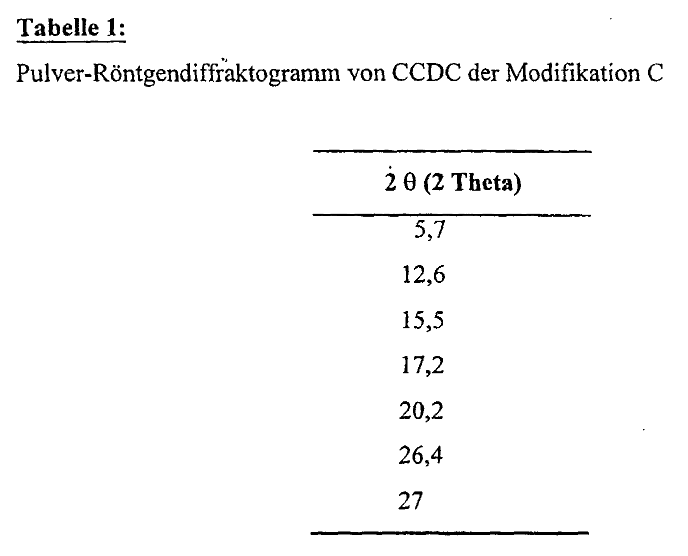

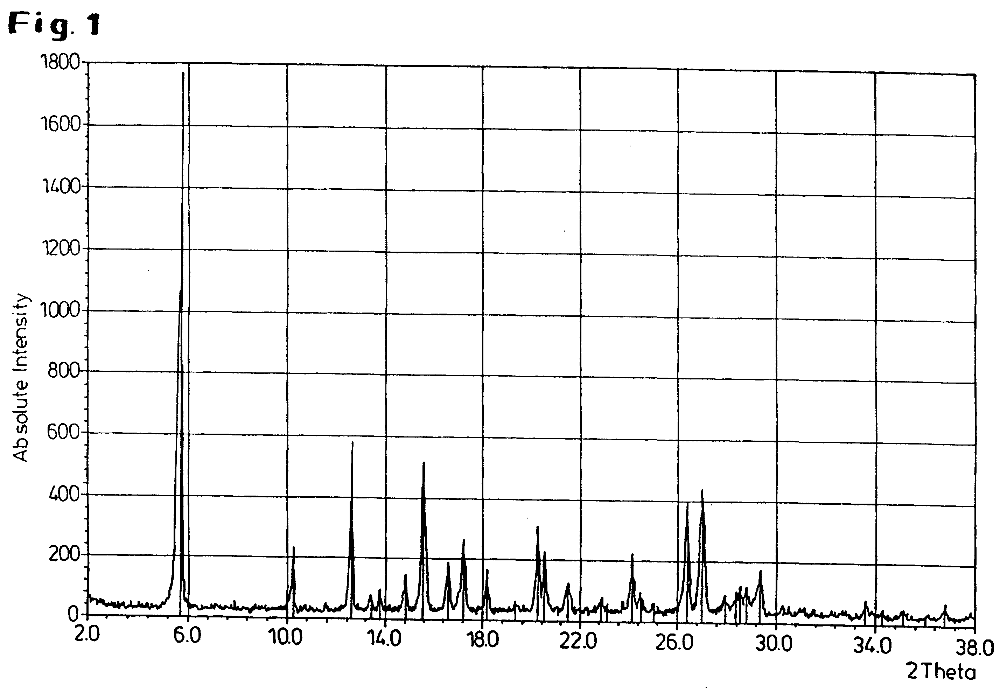

Ein charakteristisches Pulver-Röntgendiffraktogramm der Modifikation C ist auch in der Abbildung 1 wiedergegeben.

Die erfindungsgemäße Modifikation C von CCDC unterscheidet sich außerdem in einer Reihe weiterer Eigenschaften von den anderen Formen der CCDC. Diese Eigenschaften können einzeln oder gemeinsam mit den übrigen Parametern zur Charakterisierung der erfindungsgemäßen Modifikation C der CCDC dienen.

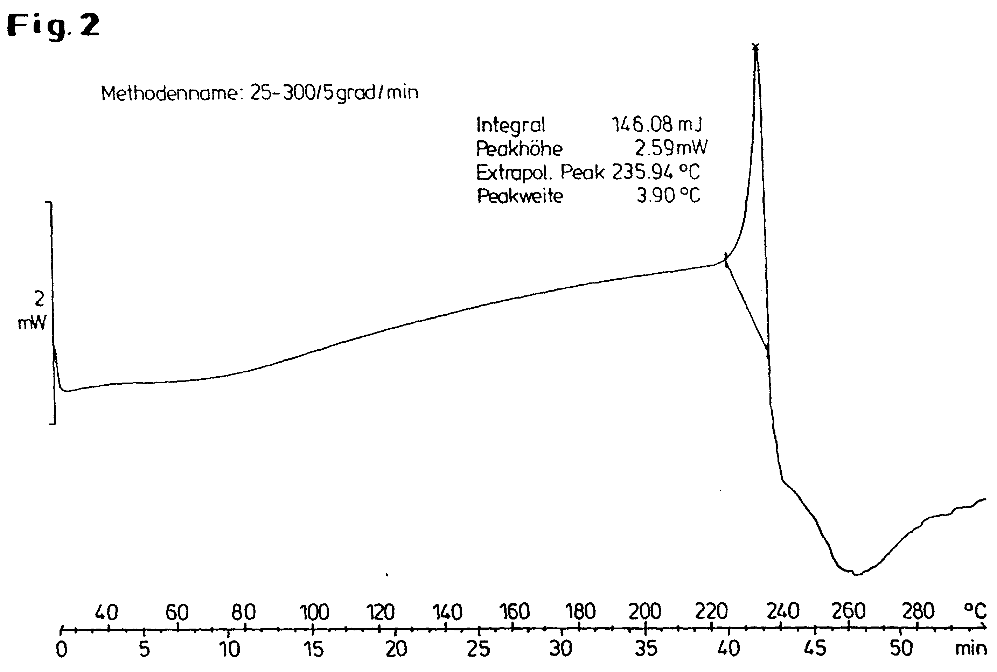

CCDC der Modifikation C ist u.a. dadurch gekennzeichnet, dass es einen mit Hilfe der Differentialthermoanalyse (DTA) bestimmten Schmelzpunkt von 235°C bis 237°C hat. Ein charakteristisches Differentialthermodiagramm ist in der Abbildung 2 wiedergegeben.

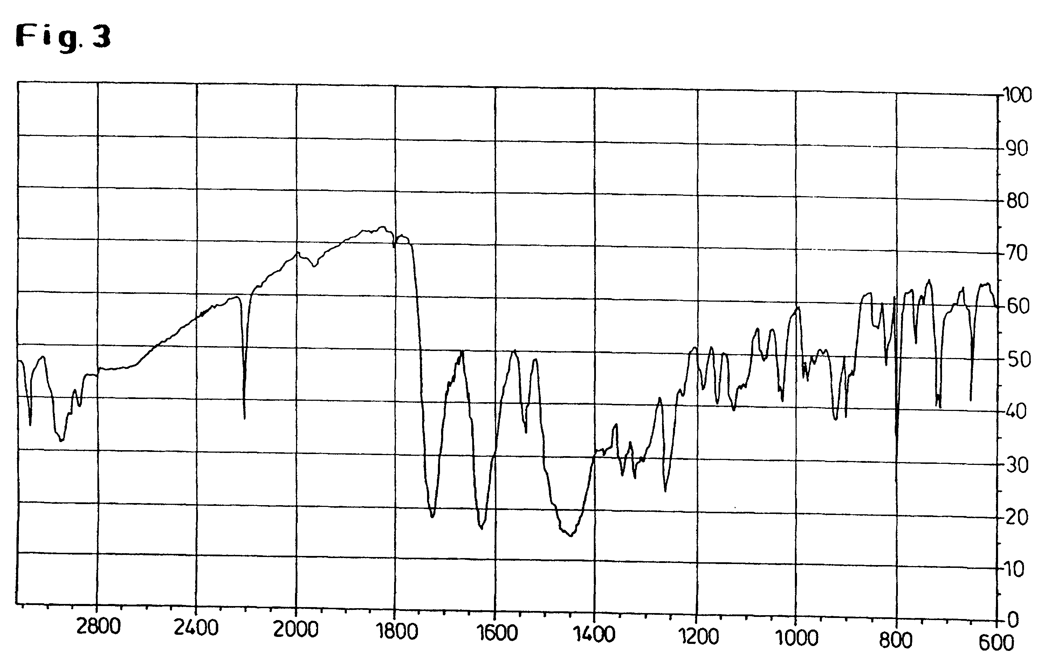

CCDC der Modifikation C ist weiterhin dadurch gekennzeichnet, dass es ein in KBr gemessenes Infrarotspektrum wie in Abbildung 3 gezeigt besitzt.

CCDC der Modifikation C ist weiterhin dadurch gekennzeichnet, dass es nach dem im folgenden angegebenen Herstellverfahren erhältlich ist. Die Kristallmodifikation C von CCDC wird dadurch erhalten, dass man CCDC unbekannter Modifikation oder amorphes CCDC mehrere Tage bei Raumtemperatur bei einer relativen Luftfeuchtigkeit von mindestens 92 % so lange lagert, bis keine Gewichtszunahme mehr erfolgt, das so erhaltene wasserhaltige Produkt trocknet und anschließend bis auf eine Temperatur, die oberhalb der Umwandlungstemperatur liegt, erhitzt.

Das Trocknen des wasserhaltigen Produktes kann nach gängigen Methoden erfolgen. So kann das wasserhaltige Produkt beispielsweise bei erhöhter Temperatur im Vakuum getrocknet werden. Es ist auch möglich, die Trocknung in Gegenwart eines üblichen Trocknungsmittels wie beispielsweise Phosphorpentoxid durchzuführen.

Die zur Umwandlung der getrockneten Probe in die Modifikation C erforderliche Temperatur kann mittels einer DTA der getrockneten Substanz bestimmt werden. In der Regel liegt sie zwischen 150°C und 180°C.

CCDC der Kristallmodifikation C ist überraschend stabil und wandelt sich auch bei längerer Lagerung nicht in eine andere Kristallmodifikation oder die amorphe Form um. Es ist aus diesen Gründen hervorragend zur Herstellung von Tabletten oder anderen Festformulierungen geeignet. Durch seine Stabilität verleiht es diesen Formulierungen die gewünschte lang andauernde Lagerstabilität. Mit der Kristallmodifikation C können damit definiert und gezielt stabile feste Zubereitungen von CCDC hergestellt werden.

CCDC der Kristallmodifikation C ist hervorragend gegen pathogene Bakterien auf dem Gebiet der Human- oder Tiermedizin wirksam. Sein breites Einsatzgebiet entspricht dem von CCDC.

Das Röntgen-Pulverdiffraktogramm zur Charakterisierung der Kristallmodifikation C von CCDC wurde mit einem Transmissions-Diffraktometer STADI-P mit ortsempfindlichem Detektor (PSD2) der Fa. Stoe erhalten.

Der Schmelzpunkt der Differentialthermoanalyse wurde mit dem Gerät DSC 820 der Fa. Mettler-Toledo erhalten. Dabei wurde die Probe von CCDC der Kristallmodifikation C in einem Aluminiumtiegel mit 10 K/min an der Luft aufgeheizt.

Das IR-Spektrum wurde mit dem Gerät 881 der Fa. Perkin-Elmer in KBr erhalten.

Die folgende Beispiele illustrieren die Erfindung ohne sie einzuschränken. Die in den folgenden Beispielen beschriebenen Verdünnungsmitteln/Basensysteme sind besonders bevorzugt.

### Vergleichsbeispiel

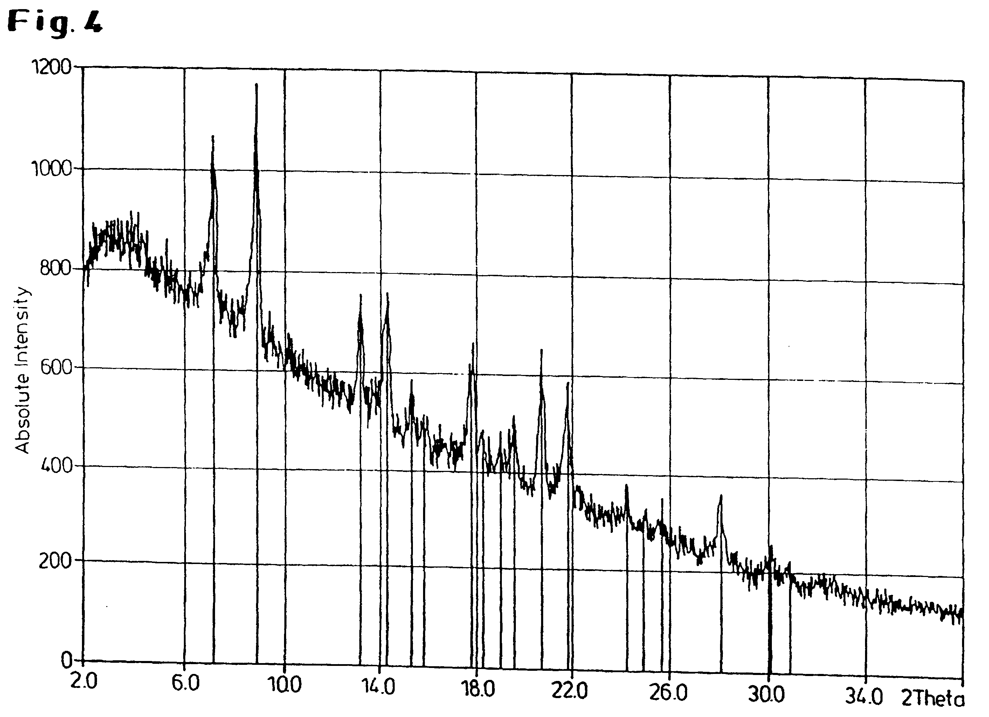

Eine Mischung aus 3,07 g 7-Chlor-8-cyan-1-cyclopropyl-6-fluor-1,4-dihydro-4-oxo-3-chinolincarbonsäure, 1,39 g (1S,6S)-2,8-Diazabicyclo[4.3.0]nonan, 2,24 g 1,4-Diazabicyclo[2.2.2]octan (DABCO), 29,5 ml Dimethylformamid und 29,5 ml Acetonitril wird 16 Stunden bei Raumtemperatur gerührt. Das Reaktionsgemisch wird bei 60°C Badtemperatur am Rotationsverdampfer eingeengt und der Rückstand in 10 ml Wasser aufgenommen. Die resultierende Lösung wird mit verdünnter Salzsäure auf pH 7 gestellt und der Feststoff abfiltriert. Das Filtrat wird dreimal mit je 20 ml Dichlormethan ausgeschüttelt. Man trocknet die organische Phase über Natriumsulfat, filtriert und engt das Filtrat am Rotationsverdampfer bei 60°C Badtemperatur ein. Man erhält 2,4 g hellbraunen Feststoff, der das in der Abbildung 4 gezeigte Röntgen-Pulverdiffraktogramm aufweist und demnach zum großen Teil amorph ist.

### Beispiel 1

1012 g 7-Chlor-8-cyan-1-cyclopropyl-6-fluor-1,4-dihydro-4-oxo-3 -chinolincarbonsäure werden in einer Mischung aus 3 300 ml Ethanol, 1 980 ml N-Methyl-pyrrolidon und 534 g Hünig-Base vorgelegt. Man erhitzt zum Rückfluss und tropft dann 459 g (1S,6S)-2,8-Diazabicyclo[4.3.0]nonan zu. Nach Beendigung des Zutropfens rührt man noch 3 Stunden unter Rückfluss, läßt dann auf Raumtemperatur abkühlen, saugt den Feststoff ab, und wäscht ihn mit insgesamt 1 800 ml Ethanol.

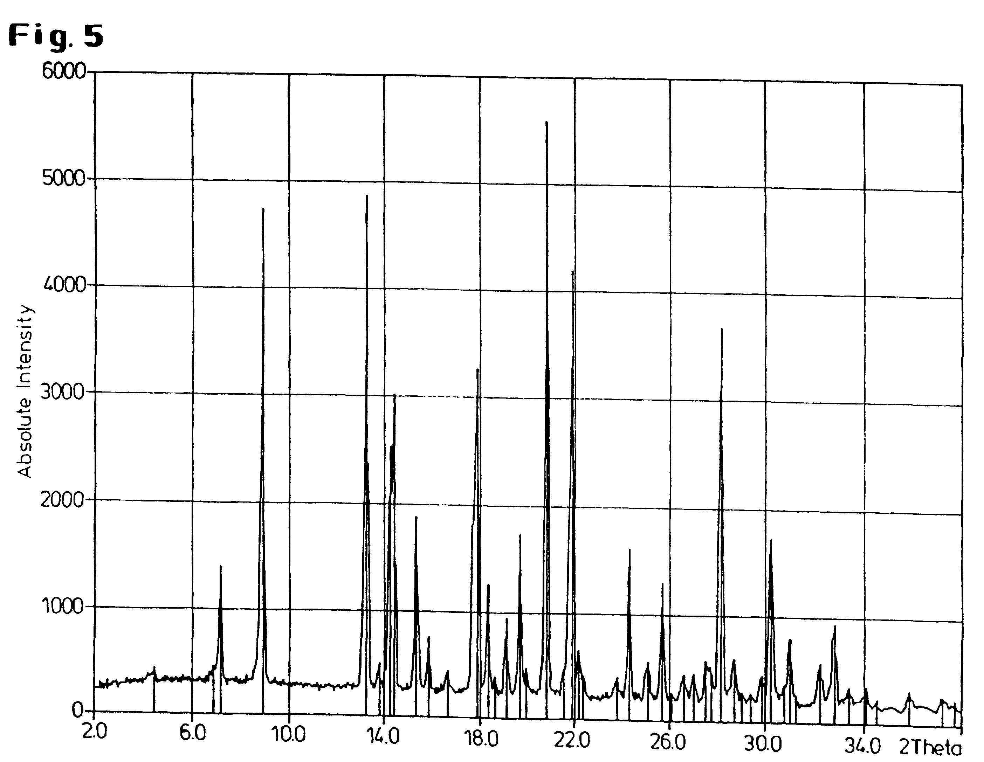

Der erhaltene Feststoff wird in einer Mischung aus 4 650 ml Ethanol und 41 g Hünig-Base suspendiert und das Reaktionsgemisch 3 Stunden zum Rückfluss erhitzt. Man läßt das Reaktionsgemisch wieder auf Raumtemperatur abkühlen, saugt den Feststoff ab, wäscht mit insgesamt 1000 ml EtOH nach und trocknet bei 60 bis 70°C im Vakuumtrockenschrank bis zur Gewichtskonstanz. Man erhält 1130 g beigen Feststoff, der das in der Abbildung 5 gezeigte Pulver-Röntgendiffraktogramm aufweist.

Eine Menge von 500 mg des gemäß dieser Vorschrift hergestellten Feststoffes wird bei Raumtemperatur 11 Tage bei einer relativen Luftfeuchtigkeit von 95 % (eingestellt durch eine gesättigte Lösung mit Bodensatz von Na₂HPO₄ x 12 H₂O in Wasser) gelagert. Man erhält 695 mg Produkt.

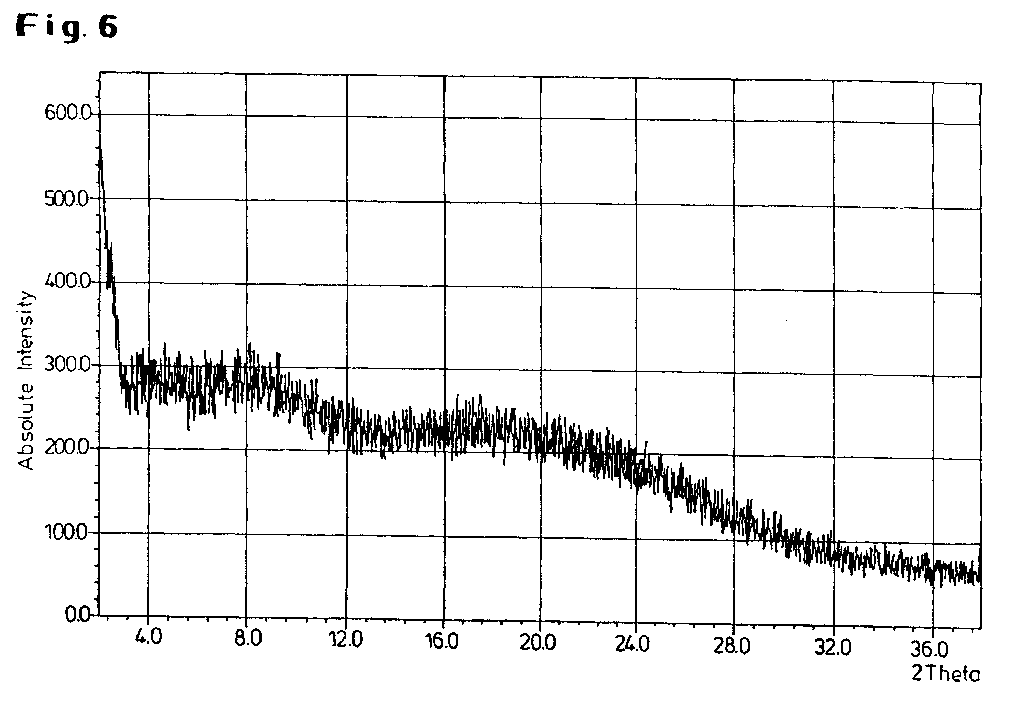

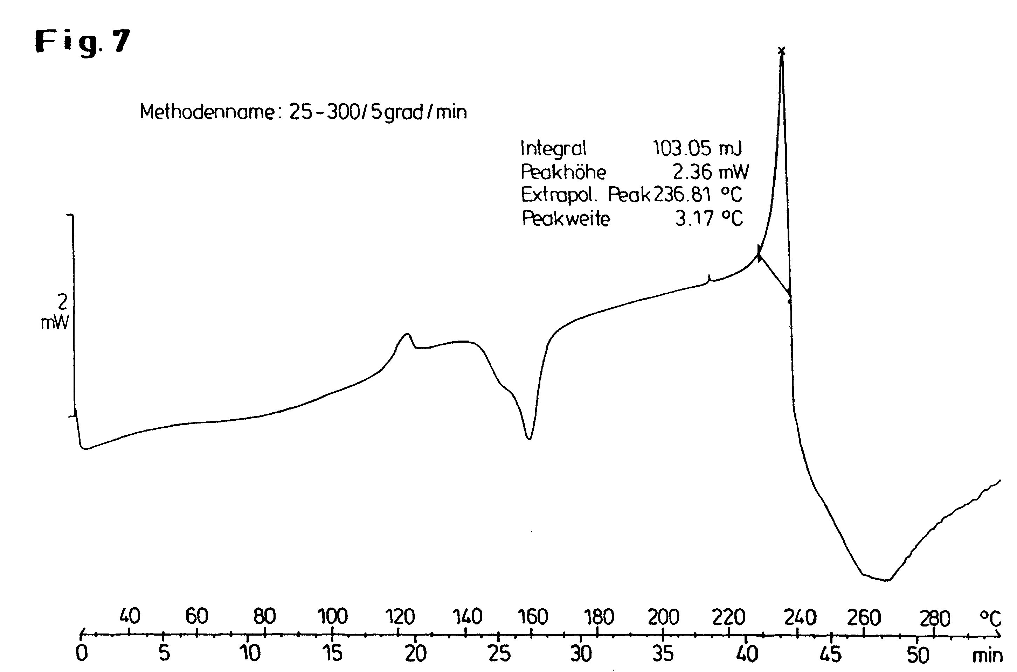

200 mg des so erhaltenen Feststoffes werden 24 Stunden bei 100°C im Vakuumtrockensckrank über P₂O₅ getrocknet. Man erhält 134 mg Feststoff, der nach Pulver-Röntgendiffraktogramm (Abb.6) weitgehend amorph ist und die in Abb. 7 gezeigte DTA aufweist.

30 mg des so erhaltenen Feststoffes werden 2 Stunden unter Stickstoff auf 180°C erhitzt. Man erhält 27 mg Feststoff, der das in der Abbildung 1 gezeigte Pulver-Röntgendiffraktogramm, das in der Abbildung 2 gezeigte Differentialthermodiagramm und das in der Abbildung 3 gezeigte IR-Spektrum aufweist.

## Patentansprüche

1. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation C, **dadurch gekennzeichnet, dass** sie ein Röntgen-Pulverdiffraktogramm mit folgenden Reflexlagen (2 Theta) hoher und mittlerer Intensität aufweist

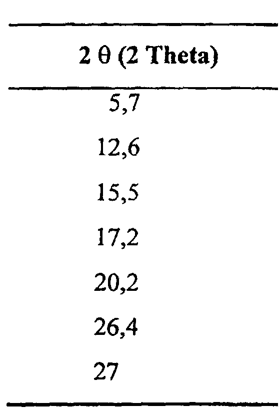

2. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation C, **dadurch gekennzeichnet, dass** sie ein Röntgen-Pulverdiffraktogramm mit folgenden Reflexlagen (2 Theta) hoher und mittlerer Intensität aufweist 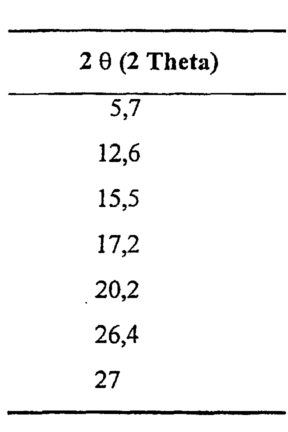 und einen durch DTA ermittelten Schmelzpunkt von 235°C bis 237°C besitzt.

3. 8-Cyan-1-cyclopropyl-7-(1S,6S-2,8-diazabicyclo[4.3.0]nonan-8-yl)-6-fluor-1,4-dihydro-4-oxo-3-chinolincarbonsäure (CCDC) der Kristallmodifikation C die ein Rontgen-Pulver-diffraktogram mit Refleklagen (2 Theta) noner und mittlerer Intensität gemäß Anspruch 1 aufweist, dadurch erhältlich, dass CCDC unbekannter Modifikation oder amorphes CCDC einer relativen Luftfeuchtigkeit von mindestens 92 % ausgesetzt wird bis keine Gewichtszunahme mehr erfolgt, das Produkt dann getrocknet und anschließend bis auf eine Temperatur, die oberhalb der Umwandlungstemperatur liegt, erhitzt wird.

4. Verfahren zur Herstellung von 8-Cyan-1-Cyclopropyl-7-(1S,6S-2,8-diazabicyclo[4.3.0], nonan-8-yl)-6-fluor-1,4-dihydro-4-oxo-3-chinolincarbonsäure (CCDC) der Kristallmodifikation C gemäß einem der Ansprüche 1 bis 3, **dadurch gekennzeichnet, dass** CCDC unbekannter Modifikation oder amorphes CCDC einer relativen Luftfeuchtigkeit von mindestens 92 % ausgesetzt wird bis keine Gewichtszunahme mehr erfolgt, das Produkt dann getrocknet und anschließend bis auf eine Temperatur, die oberhalb der Umwandlungstemperatur liegt, erhitzt wird.

5. Arzneimittel, **dadurch gekennzeichnet, dass** es neben üblichen Hilfs- und Trägerstoffen 8-Cyan-1-Cyclopropyl-7-(1S,6S-2,8-diazabicyclo[4.3.0], nonan-8-yl)-6-fluor-1,4-dihydro-4-oxo-3-chinolincarbonsäure (CCDC) der Modifikation C gemäß einem der Ansprüche 1 bis 3 enthält.

6. Verwendung von 8-Cyan-1-Cyclopropyl-7-(1S,6S-2,8-diazabicyclo [4.3.0]nonan-8-yl)-6-fluor-1,4-dihydro-4-oxo-3-chinolincarbonsäure (CCDC) der Modifikation C gemäß einem der Ansprüche 1 bis 3 zur Herstellung von Arzneimitteln.

7. 8-Cyan-1-Cyclopropyl-7-(1S,6S-2,8-diazabicyclo [4.3.0]nonan-8-yl)-6-fluor-1,4-dihydro-4-oxo-3-chinolincarbonsäure (CCDC) der Modifikation C gemäß einem der Ansprüche 1 bis 3 zur Verwendung als antibakterielles Mittel.

## Claims

1. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification C, **characterized in that** it has an X-ray powder diffractogram with the following reflection signals (2 theta) of high and medium intensity

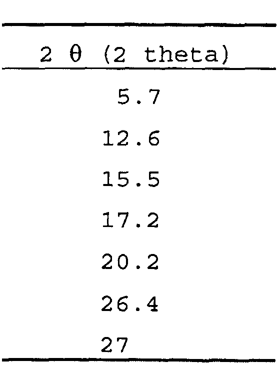

2. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification C, **characterized in that** it has an X-ray powder diffractogram with the following reflection signals (2 theta) of high and medium intensity 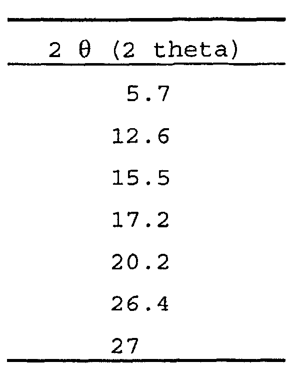 and a melting point, determined by DTA, of from 235°C to 237°C.

3. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification C, which has an X-ray powder diffractogram with reflex signals (2 theta) of high and medium intensity according to Claim 1, obtainable by subjecting CCDC of unknown modification or amorphous CCDC to a relative atmospheric humidity of at least 92% until there is no further weight increase, followed by drying of the product and subsequent heating to a temperature above the rearrangement temperature.

4. Process for preparing 8-cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the crystal modification C according to any of Claims 1 to 3, **characterized in that** CCDC of unknown modification or amorphous CCDC is subjected to a relative atmospheric humidity of at least 92% until there is no further weight increase, followed by drying of the product and subsequent heating to a temperature above the rearrangement temperature.

5. Medicament, **characterized in that** it comprises, in addition to customary auxiliaries and excipients, 8-cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the modification C according to one of Claims 1 to 3.

6. Use of 8-cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the modification C according to one of Claims 1 to 3 for preparing medicaments.

7. 8-Cyano-1-cyclopropyl-7-(1S,6S-2,8-diazabicyclo[4.3.0]nonan-8-yl)-6-fluoro-1,4-dihydro-4-oxo-3-quinolinecarboxylic acid (CCDC) of the modification C according to one of Claims 1 to 3 for use as an antibacterial composition.

## Revendications

1. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C, **caractérisé en ce qu'**il présente un diffractogramme des rayons X sur poudre ayant les positions de réflexion (2 thêta) suivantes de haute et moyenne intensités :

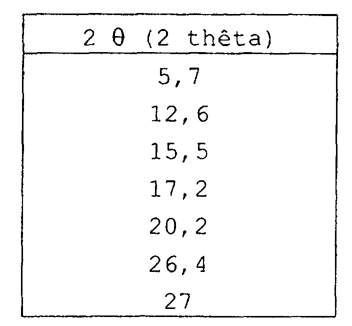

2. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C, **caractérisé en ce qu'**il présente un diffractogramme des rayons X sur poudre avec les positions de réflexion (2 thêta) de haute et moyenne intensité suivantes : 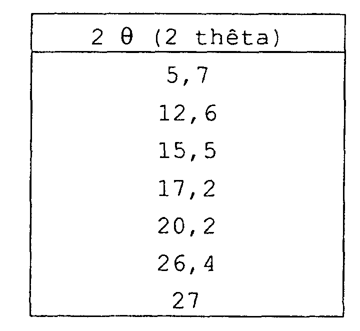 et un point de fusion, déterminé par thermo-analyse différentielle, de 235 à 237°C.

3. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C, qui présente un diffractogramme des rayons X sur poudre avec les positions de réflexion (2 thêta) de haute et moyenne intensités suivant la revendication 1, pouvant être obtenu par le fait qu'on expose une forme inconnue de CCDC ou du CCDC amorphe à une humidité atmosphérique relative d'au moins 92 % jusqu'à ce qu'il n'y ait plus de gain de poids, on sèche ensuite le produit, puis on le chauffe jusqu'à une température qui se trouve au-dessus de la température de transformation.

4. Procédé de production d'acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C suivant l'une des revendications 1 à 3, **caractérisé en ce qu'**on expose une forme inconnue de CCDC ou du CCDC amorphe à une humidité atmosphérique relative d'au moins 92 % jusqu'à ce qu'il n'y ait plus de gain de poids, on sèche ensuite le produit, puis on le chauffe jusqu'à une température qui est au-dessus de la température de transformation.

5. Médicament, **caractérisé en ce qu'**il contient, à côté de substances auxiliaires et de supports classiques, de l'acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C suivant l'une des revendications 1 à 3.

6. Utilisation de l'acide 8-cyano-1-cyclopropyl-7-(13,6S-2,8-diazabicyclo[4.3.0]nonane-8-yl)-6-fluoro-1,4-dihydro-4-oxo-3-quinoléinecarboxylique (CCDC) de la forme cristalline C suivant l'une des revendications 1 à 3 pour la préparation de médicaments.

7. Acide 8-cyano-1-cyclopropyl-7-(1S,6S-2,8-diazabicyclo[4.3.0]nonane-8-yl)-6-fluoro-1,4-dihydro-4-oxo-3-quinoléinecarboxylique (CCDC) de la forme cristalline C suivant l'une des revendications 1 à 3, destiné à être utilisé comme agent antibactérien.
